# EUROPÄISCHE PATENTSCHRIFT

(11) **EP 1 079 750 B1**
(45) Veröffentlichungstag und Bekanntmachung des Hinweises auf die Patenterteilung: **02.08.2006**
(21) Anmeldenummer: 98919021.0
(22) Anmeldetag: 19.05.1998
(51) Int. Cl.: A61B 17/64

(54) **VERBINDUNGSELEMENT FÜR MONOLATERALES EXTERNES FIXATIONSSYSTEM FÜR TRAUMATOLOGIE UND ORTHOPÄDIE**
CONNECTING ELEMENT FOR A MONOLATERAL EXTERNAL FIXATION SYSTEM FOR TRAUMATOLOGY AND ORTHOPAEDICS
ELEMENT DE LIAISON POUR SYSTEME DE FIXATION EXTERNE MONOLATERAL DE TRAUMATOLOGIE ET D'ORTHOPEDIE

(43) Veröffentlichungstag der Anmeldung: 07.03.2001
(73) Patentinhaber: Synthes AG Chur, 7002 Chur (CH)
(72) Erfinder: MARTINELLI, Orlando, CH-3018 Bern (CH); INAUEN, Beat, CH-4434 Hölstein (CH); FLÜHLER, Erwin, CH-4123 Allschwil (CH); CLAES, Lutz, D-89233 Neu-Ulm (DE); GERNGROSS, Heinz, D-89075 Ulm (DE); RÜBSAAMEN, Götz, D-83278 Traunstein (DE)
(74) Vertreter: Lusuardi, Werther
(86) Internationale Anmeldenummer: PCT/CH1998/000206
(87) Internationale Veröffentlichungsnummer: WO 1999/059489

(56) Entgegenhaltungen:
- WO-A-83/02554
- WO-A-91/11149
- DE-U- 9 319 433

## Beschreibung

Die Erfindung bezieht sich auf eine Vorrichtung zum äusseren Festlegen von Teilen von gebrochenen Knochen gemäss dem Oberbegriff des Patentanspruchs 1.

Aus dem Stand der Technik ist aus der EP 0 011 258 Al ORTHOFIX eine Vorrichtung zum äusseren Festlegen der Teile eines gebrochenen Knochens bekannt. Diese bekannte Erfindung umfasst einen langgestreckten Mittelkörper, der aus zwei parallel zu seiner Längsachse gegeneinander verschiebbaren Teilen besteht, wobei jeder Teil des Mittelkörpers eine Klemmeinrichtung für in einen Knochenteil einsetzbare Nägel oder Knochenschrauben trägt, und eine an den beiden Teilen des Mittelkörpers angreifende Druck- und Zugeinrichtung. Die Klemmeinrichtungen sind mittels Kugelgelenken an den Enden der verschiebbaren Teile des Mittelkörpers gelagert, was eine Schwenkbarkeit der Klemmeinrichtungen und der in die Knochenteile einsetzbaren Nägel oder Schrauben mit drei Freiheitsgraden ermöglicht.

Eine weitere aus dem Stand der Technik bekannte Vorrichtung zur externen Fixierung von Knochen mittels Stiften ist in der US 5,160,335 WAGENKNECHT offenbart. Diese bekannte Erfindung umfasst einen Fixationsstab, der auch teleskopierbar sein kann, mit Klemmelementen zum Festhalten der Knochenstifte, Verbindungsteile mit gekrümmten Auflageflächen für die Klemmelemente und Klammern zur Befestigung der Klemmelemente mit den Verbindungsteilen auf dem Fixationsstab. Die Verbindungsteile sind so gestaltet, dass die Klemmelemente gegenüber den Klammern mit drei Freiheitsgraden schwenkbar sind.

Beide aus dem Stand der Technik bekannten Vorrichtungen zeigen den Nachteil, dass der Fixationsstab zwar teleskopierbar ist aber die Bewegung der zu teleskopierenden Teile durch eine extern am Fixationsstab angebrachte Druck- und Zugeinrichtung beziehungsweise durch eine am Fixationsstab angebrachte Feststellschraube verursacht wird. Zudem ist bei beiden bekannten Vorrichtungen der Fixationsstab nur auf einer Seite verlängerbar und der Fixationsstab weist auf seiner gesamten Länge nicht den gleichen Durchmesser auf, so dass die Backen nicht an einer beliebigen Stelle auf dem Fixationsstab montiert werden können.

Hier will die Erfindung Abhilfe schaffen. Der Erfindung liegt die Aufgabe zugrunde, ein Verbindungselement zu gestalten, das intern und beidseitig verlängerbar ist. Damit werden doppelseitige Verlängerungen möglich, wodurch die Behandlungszeit halbiert werden könnte. Auch soll die Steifigkeit des Verbindungselementes durch die Ersetzbarkeit der verschiedenen Teile durch Teile, welche aus verschiedenen Materialien bestehen, wählbar sein.

Die Erfindung löst die gestellte Aufgabe mit einer Vorrichtung zum äusseren Festlegen von Teilen von gebrochenen Knochen, welche die Merkmale des Anspruchs 1 aufweist.

Weitere vorteilhafte Ausgestaltungen der Erfindung sind in den abhängigen Ansprüchen gekennzeichnet.

In einer bevorzugten Ausführungsform der erfindungsgemässen Vorrichtung umfasst diese ein longitudinales Verbindungselement mit einer Zentralachse. Das Verbindungselement ist in Richtung dieser Zentralachse beidseitig verdrehfest teleskopierbar und es lassen sich auf dem Verbindungselement Backen zur Fixierung von Knochenschrauben oder Stiften lösbar anbringen. Das Verbindungselement umfasst ein Mittelteil und zwei entlang der Zentralachse verschiebbare Aussenteile. Mittels der verschiebbaren Aussenteile ist das Verbindungselement beidseitig verlängerbar und weist auf seiner Länge den selben Durchmesser auf. Die Auswechselbarkeit des Mittelteils respektive der Aussenteile ermöglicht eine wählbare Steifigkeit des Verbindungselementes.

Wiederum eine andere Ausführungsform der erfindungsgemässen Vorrichtung unterscheidet sich von der vorangehend beschriebenen Ausführungsform dadurch, dass das Verbindungselement aus einem zylindrischen Mittelteil und zwei konzentrischen hohlzylindrischen Aussenteilen besteht. Die Aussenteile sind auf dem Mittelteil axial gleitbar gelagert und verdrehfest teleskopierbar.

Wiederum eine weitere Ausführungsform der erfindungsgemässen Vorrichtung unterscheidet sich von den vorangehend beschriebenen Ausführungsformen nur darin, dass der Querschnitt des Mittelteiles eine polygonförmige Aussenform aufweist und der Querschnitt der Aussenteile eine zu dieser polygonförmigen Aussenform des Mittelteiles korrespondierende polygonförmige Innenform aufweist. Auch bei dieser Ausführungsform sind die Aussenteile auf dem Mittelteil axial gleitbar gelagert. Die Teleskopierbarkeit, ohne dass sich die Aussenteile gegeneinander oder gegenüber dem Mittelteil verdrehen können, ist durch die polygonförmige Aussenform des Mittelteiles und durch die ebenfalls polygonförmige Innenform der Aussenteile gewährleistet.

Eine andere Ausführungsform der erfindungsgemässen Vorrichtung unterscheidet sich von den vorangehend beschriebenen Ausführungsformen nur darin, dass die äussere Mantelfläche des Mittelteiles als Keilwelle ausgebildet ist und die Aussenteile im Querschnitt eine dazu korrespondierende Innenform aufweisen.

Eine weitere Ausführungsform der erfindungsgemässen Vorrichtung unterscheidet sich von den vorangehend beschriebenen Ausführungsformen nur darin, dass die axiale Position der Aussenteile relativ zum Mittelteil durch Transportspindeln verstellbar und fixierbar ist. Die Transportspindeln sind von den äusseren Enden des Verbindungselementes bedienbar und gestatten eine ein- bzw. beidseitige Verlängerung des Verbindungselementes, ohne dass sich die Transportspindeln gegenseitig beeinflussen.

Wiederum eine weitere Ausführungsform der erfindungsgemässen Vorrichtung unterscheidet sich von der vorangehend beschriebenen Ausführungsform darin, dass die Transportspindeln durch in die Aussenteile einschraubbare Endstücke blockierbar sind.

Eine andere Ausführungsform der erfindungsgemässen Vorrichtung unterscheidet sich von der vorangehend beschriebenen Ausführungsform dadurch, dass eines der Endstücke ein Dynamisierungsdeckel ist und durch ein graduelles Herausschrauben dieses Dynamisierungsdeckels die axiale Einspannung der Gewindespindel millimeterweise freigegeben werden kann, wodurch das Aussenteil axial um einen kontrollierbaren Betrag verschieblich ist und dadurch eine Dynamisierung der Fraktur erreicht werden kann.

Weitere Ausführungsformen der erfindungsgemässen Vorrichtung unterscheiden sich von den vorangehend beschriebenen Ausführungsformen nur darin, dass durch die Materialwahl des Mittelteiles und der Aussenteile die Steifigkeit des Verbindungselementes wählbar ist. Folgende Materialien sind für das Mittelteil, für das Mittelteil mit der Transporteinrichtung, welche sich beispielsweise durch ein Transportgewinde realisieren lässt, und für die Aussenteile denkbar:
- Aluminium
- Stahl
- Kohlefasern
- Titan

Damit lassen sich auch Kombinationen von verschiedenen Materialien für das Mittelteil und die Aussenteile herstellen, womit die Steifigkeit des Verbindungselementes beeinflusst werden kann.

Wiederum eine andere Ausführungsform der erfindungsgemässen Vorrichtung unterscheidet sich von den vorangehend beschriebenen Ausführungsformen nur darin, dass sich auf dem Mittelteil zwischen den Aussenteilen ein Zwischenelement einsetzen lässt, welches auf dem Mittelteil verschiebbar und verdrehfest ist. Zudem weist dieses Zwischenelement den selben Aussendurchmesser auf wie die Aussenteile, wodurch auch auf diesem Zwischenelement eine oder mehrere Backen zur Fixierung von Knochenschrauben oder Stiften lösbar angebracht werden können. Ein entlang der Längsachse des Zwischenelementes durchgehender Schlitz im Zwischenelement ermöglicht, dass das so geschlitzte Zwischenelement durch die Klemmkraft einer auf dem Zwischenelement angebrachten Backe auf dem Mittelteil fixiert wird. Anstelle eines einzigen Schlitzes kann das Zwischenelement auch zwei auf zwei gegenüber liegenden Seiten angebrachte Schlitze aufweisen.

Die durch die Erfindung erreichten Vorteile sind im wesentlichen darin zu sehen, dass das Verbindungselement intern beidseitig verlängerbar ist und auf seiner ganzen Länge mit Ausnahme eines kleinen Mittelstückes den gleichen Aussendurchmesser aufweist. Dadurch können die Backen, welche zur Aufnahme und Fixierung der Knochenschrauben dienen, an beliebiger Stelle auf dem Verbindungselement angebracht werden. Ein zusätzliches Zwischenelement ermöglicht die Plazierung einer oder mehrerer Backen auch an einer Stelle zwischen den Aussenteilen. Zudem lassen sich Mittelteil und Aussenteile, welche aus verschiedenen Materialien bestehen, zusammenfügen, wodurch die Steifigkeit des Verbindungselementes beeinflussbar ist.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Aufriss einer Ausführungsform der erfindungsgemässen Vorrichtung mit zwei Backen zur Fixierung von Knochenschrauben;
Fig. 2 einen Längsschnitt durch das Verbindungselement einer Ausführungsform der erfindungsgemässen Vorrichtung; und
Fig. 3. eine Teilansicht einer Ausführungsform der erfindungsgemässen Vorrichtung.

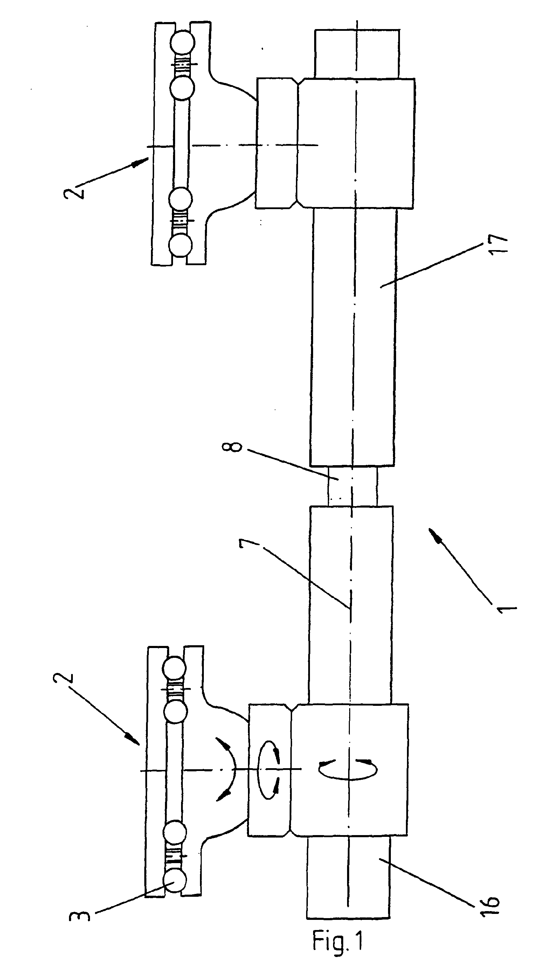

Fig 1. zeigt eine Ausführungsform der erfindungsgemässen Vorrichtung mit einem Verbindungselement 1 mit der Zentralachse 7. Koaxial zur Zentralachse 7 angeordnet sind die hohlzylindrischen Aussenteile 16;17 und das Mittelteil 8. Die Aussenteile 16;17 sind so mit Bohrungen ausgestattet, dass sie auf dem Mittelteil 8 gleitbar sind und in Richtung der Zentralachse verschoben werden können, wodurch das Verbindungselement in beiden Richtungen teleskopierbar wird. Auf den Aussenteilen 16;17 lösbar befestigt sind Backen 2 zur Aufnahme und Fixierung von Knochenschrauben 3. Die Backen 2 können entlang der Zentralachse 7 auf den Aussenteilen 16;17 verschoben werden und bei Bedarf können auch weitere Backen 2 eingefügt werden.

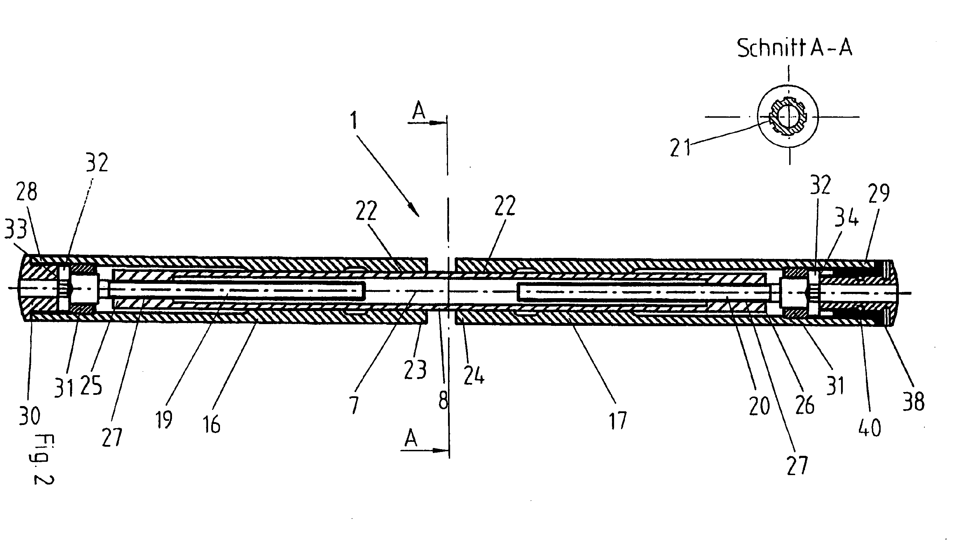

In Fig. 2 ist ein Längsschnitt durch das Verbindungselement 1 einer Ausführungsform der erfindungsgemässen Vorrichtung dargestellt. Das Verbindungselement 1 umfasst als teleskopierbare Elemente das Mittelteil 8 und die auf diesem Mittelteil 8 gleitbaren Aussenteile 16;17, wobei das Mittelteil 8 und die Aussenteile 16;17 hohlzylindrisch ausgebildet sind. Die äussere Mantelfläche 21 des Mittelteiles 8 ist als Keilwelle ausgebildet. Zu dieser Keilwelle korrespondierend sind die Bohrungen 22 an den inneren Enden 23;24 der Aussenteile 16;17 ausgebildet, so dass die Aussenteile 16;17 verdrehfest teleskopierbar sind. An den äusseren axialen Enden 25;26 des Mittelteils 8 sind Innengewinde 27 angebracht, in welche die zur axialen Verschiebung der Aussenteile 16;17 vorgesehenen Gewindespindeln 19;20 ein- beziehungsweise ausgeschraubt werden können, wodurch die axiale Länge des Verbindungselementes 1 festgelegt wird. Zur axialen Fixierung der Gewindespindeln 19;20 sind in den Aussenteilen 16;17 an deren äusseren Enden 28; 29 Hülsen 31 und Endstücke 30;38 in die Bohrungen der Aussenteile 16;17 so eingefügt, dass die Borde 32, die an den einen Enden 33,34 der Gewindespindeln 19;20 angebracht sind, zwischen Hülsen 31 und Endstücken 30;38 axialfest eingespannt sind. Soll eine Dynamisierung der Fraktur erreicht werden, kann durch ein graduelles Herausschrauben des Endstückes (38) die axiale Einspannung der Gewindespindel (20) millimeterweise freigegeben werden. Dadurch wird das Aussenteil (17) axial um einen kontrollierbaren Betrag verschieblich. Durch den elastischen Ring (40) wird das als Dynamisierungsdeckel verwendete Endstück (38) in einer einstellbaren Lage gehalten und vor dem Herausfallen geschützt.

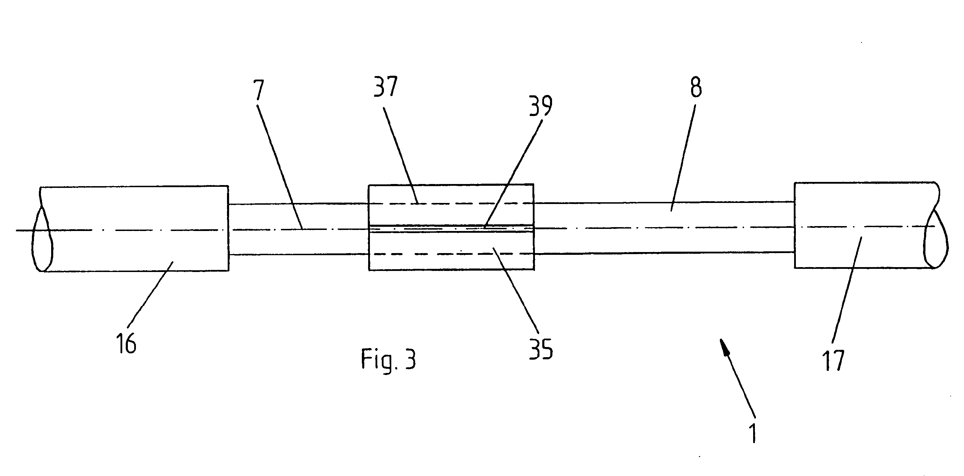

In Fig. 3 ist eine Ausführungsform der erfindungsgemässen Vorrichtung dargestellt, die sich von den in Fig. 1 und 2 darin unterscheidet, dass auf dem Mittelteil 8 zwischen den auseinander gefahrenen Aussenteilen 16;17 zusätzlich ein Zwischenelement 35 angeordnet ist. Dieses Zwischenelement 35 ist auf dem Mittelteil 8 gleitbar und mittels einer zu der Keilwellenausführung des Mittelteils 8 korrespondierenden Bohrung 37 verdrehfest angeordnet. Das Zwischenelement 35 weist auf einer Seite entlang der Zentralachse (7) einen durchgehenden Schlitz (39) auf. Die Fixierung der axialen Position des Zwischenelementes 35 erfolgt über die Klemmkraft der Backen 2, wodurch das Zwischenelement 35 zusammen gepresst und auf dem Mittelteil (8) festgeklemmt wird.

## Patentansprüche

1. Vorrichtung zum äusseren Festlegen von Teilen von gebrochenen Knochen, welche ein longitudinales Verbindungselement (1) mit einer Zentralachse (7) umfasst, wobei
A) das Verbindungselement (1) in Richtung dieser Zentralachse (7) verdrehfest teleskopierbar ist;
B) sich auf dem Verbindungselement (1) Backen (2) zur Fixierung von Knochenschrauben (3) lösbar anbringen lassen;
C) das Verbindungselement (1) ein Mittelteil (8) und zwei entlang der Zentralachse (7) auf dem Mittelteil (8) verschiebbare Aussenteile (16;17) umfasst; und
D) das Verbindungselement (1) mittels der Aussenteile (16;17), welche an den entgegengesetzten Enden (25;26) des Mittelteils (8) aus- und einfahrbar sind, beidseitig verlängerbar ist;
**dadurch gekennzeichnet, dass**
E) die axiale Position der Aussenteile (16;17) relativ zum Mittelteil (8) durch Transportmittel (19;20), welche innerhalb der Aussenteile (16;17) angeordnet sind, verstellbar und fixierbar ist.

2. Vorrichtung nach Anspruch 1, **dadurch gekennzeichnet, dass** das Mittelteil (8) einerseits und die Aussenteile (16;17) andererseits aus verschiedenen Werkstoffen bestehen und jedes Teil (8;16;17) auswechselbar ist und damit die Steifigkeit des Verbindungselementes (1) insgesamt durch Zusammenbau von aus verschiedenen Werkstoffen bestehenden Teilen (8;16;17) steuerbar ist.

3. Vorrichtung nach Anspruch 1 oder 2, **dadurch gekennzeichnet, dass** die Aussenteile (16;17) auf ihrer gesamten Länge den gleichen Durchmesser aufweisen.

4. Vorrichtung nach einem der Ansprüche 1 bis 3, **dadurch gekennzeichnet, dass** die Aussenteile (16;17), welche an den entgegengesetzten Enden (25;26) des Mittelteils (8) angeordnet sind, unabhängig voneinander aus- und einfahrbar sind.

5. Vorrichtung nach einem der Ansprüche 1 bis 4, **dadurch gekennzeichnet, dass** die äussere Mantelfläche des Mittelteiles (8) als Keilwelle ausgebildet ist und die Aussenteile (16;17) im Querschnitt eine dazu korrespondierende Innenform aufweisen.

6. Vorrichtung nach einem der Ansprüche 1 bis 5, **dadurch gekennzeichnet, dass** die Transportmittel (19;20) Transportspindeln sind, welche in den Mittelteil (8) ein- und ausschraubbar sind.

7. Vorrichtung nach Anspruch 6, **dadurch gekennzeichnet, dass** die als Transportspindeln ausgebildeten Transportmittel (19;20) durch in die Aussenteile (16;17) einschraubbare Endstücke (30;38) blockierbar sind.

8. Vorrichtung nach Anspruch 7, **dadurch gekennzeichnet, dass** mindestens ein in die Aussenteile (16;17) einschraubbares Endstück (30;38) als Dynamisierungsdeckel mit einem elastischen Ring (40) ausgebildet ist und die Dynamisierung darin besteht, dass durch ein graduelles Herausschrauben des mindestens einen Endstückes (30;38) die axiale Einspannung mindestens eines Transportmittels (19;20) millimeterweise freigegeben werden kann, wodurch mindestens ein Aussenteil (16;17) axial um einen kontrollierbaren Betrag verschieblich ist und **dadurch** eine Dynamisierung der Fraktur erreicht werden kann.

9. Vorrichtung nach einem der Ansprüche 1 bis 8, **dadurch gekennzeichnet, dass** sich auf dem Mittelteil (8) zwischen den Aussenteilen (16;17) mindestens ein Zwischenelement (35) einsetzen lässt, welches auf dem Mittelteil (8) verschiebbar und verdrehfest ist und auf dem Mittelteil (8) in einer gewünschten Position fixierbar ist.

10. Vorrichtung nach Anspruch 9, **dadurch gekennzeichnet, dass** auf dem äusseren Umfang des Zwischenelementes (35) mindestens eine Backe (2) aufsetzbar ist.

11. Vorrichtung nach Anspruch 9 oder 10, **dadurch gekennzeichnet, dass** das Zwischenelement (35) auf einer Seite entlang der Zentralachse (7) einen durchgehenden Schlitz (39) aufweist.

12. Vorrichtung nach Anspruch 9 oder 10, **dadurch gekennzeichnet, dass** das Zwischenelement (35) auf zwei einander gegenüber liegenden Seiten entlang der Zentralachse (7) einen durchgehenden Schlitz (39) aufweist.

13. Vorrichtung nach einem der Ansprüche 9 bis 12, **dadurch gekennzeichnet, dass** das Zwischenelement (35) durch die Klemmkraft der Backe (2) zusammenpressbar und somit auf dem Mittelteil (8) fixierbar ist.

14. Vorrichtung nach einem der Ansprüche 1 bis 13, **dadurch gekennzeichnet, dass** die Aussenteile (16;17) aus Aluminium gefertigt sind.

15. Vorrichtung nach einem der Ansprüche 1 bis 13, **dadurch gekennzeichnet, dass** die Aussenteile (16;17) aus Stahl gefertigt sind.

16. Vorrichtung nach einem der Ansprüche 1 bis 13, **dadurch gekennzeichnet, dass** die Aussenteile (16;17) aus einem Kohlefasermaterial gefertigt sind.

17. Vorrichtung nach einem der Ansprüche 1 bis 13, **dadurch gekennzeichnet, dass** die Aussenteile (16;17) aus Titan gefertigt sind.

18. Vorrichtung nach einem der Ansprüche 1 bis 17, **dadurch gekennzeichnet, dass** das Mittelteil (8) aus Aluminium gefertigt ist.

19. Vorrichtung nach einem der Ansprüche 1 bis 17, **dadurch gekennzeichnet, dass** das Mittelteil (8) aus Stahl gefertigt ist.

20. Vorrichtung nach einem der Ansprüche 1 bis 17, **dadurch gekennzeichnet, dass** das Mittelteil (8) aus einem Kohlefasermaterial gefertigt ist.

21. Vorrichtung nach einem der Ansprüche 1 bis 17, **dadurch gekennzeichnet, dass** das Mittelteil (8) aus Titan gefertigt ist.

22. Vorrichtung nach einem der Ansprüche 1 bis 21, **dadurch gekennzeichnet, dass** die koaxial zur Zentralachse (7) verlaufenden Aussenflächen der Aussenteile (16;17) rotationssymmetrisch sind.

23. Vorrichtung nach einem der Ansprüche 1 bis 22, **dadurch gekennzeichnet, dass** das Mittelteil (8) im Querschnitt eine polygonförmige Aussenform aufweist und die Aussenteile (16;17) im Querschnitt eine zu dieser polygonförmigen Aussenform korrespondierende polygonförmige Innenform aufweisen.

## Claims

1. Device for the external fixation of fragments of fractured bones, said device comprising a longitudinal connecting element (1) with a centre axis (7), whereby
A) the connecting element (1) is telescopable in the direction of said centre axis (7) in rotationally immobile fashion;
B) fastening jaws (2) can be detachably mounted on the connecting element (1) for fastening bone screws (3);
C) the connecting element (1) incorporates a central member (8) as well as two outer parts (16, 17) designed to be movable along the centre axis (7) on said central member (8); and
D) by means of the outer parts (16, 17) which can be moved in and out at the opposite ends (25, 26) of the central member (8), the connecting element (1) is bilaterally extensible; **characterized in that**
E) the axial position of the outer parts (16, 17) relative to the central member (8) is both adjustable and lockable by means of drive elements (19, 20) provided inside the outer parts (16, 17).

2. Device as in claim 1, **characterized in that** the central member (8) and, respectively, the outer parts (16, 17) consist of different materials and that each part (8, 16, 17) is exchangeable so as to permit controlling the degree of rigidity of the connecting element (1) as a whole by assembling it from components (8, 16, 17) consisting of different materials.

3. Device as in claim 1 or 2, **characterized in that** the outer parts (16, 17) are of a uniform diameter over their entire length.

4. Device as in one of the claims 1 to 3, **characterized in that** the outer parts (16, 17) which are provided at opposite ends (25, 26) of the central member (8), can be moved in and out independent of one another.

5. Device as in one of the claims 1 to 4, **characterized in that** the outer surface of the central member (8) is in the form of a keyed shaft and that the outer parts (16, 17) feature a correspondingly matching inside cross section.

6. Device as in one of the claims 1 to 5, **characterized in that** the said drive elements (19, 20) are threaded drive spindles which can be screwed into and out of the central member (8).

7. Device as in claim 6, **characterized in that** the drive spindles serving as the drive elements (19, 20) are positionally lockable by means of terminal elements (30, 38) which can be screwed into the outer parts (16, 17).

8. Device as in claim 7, **characterized in that** at least one of the terminal elements (30, 38) screwed into the outer parts (16, 17) is in the form of a dynamic-adjustment end cap with an elastic collar (40), the dynamic adjustment consisting in the progressive unscrewing of at least one such end cap (30, 38) which permits the millimeter-by-millimeter relaxation of the axial clamping of at least one drive element (19, 20) and thus the axial movement of at least one outer part (16, 17) by a controllable amount so as to achieve a dynamic adjustment of the fracture.

9. Device as in one of the claims 1 to 8, **characterized in that** at least one intermediate element (35) can be positioned on the central member (8) between the outer parts (16, 17) in such manner that it is axially movable on the central member (8) in rotationally immobile fashion and can be locked in a given axial position on the central member (8).

10. Device as in claim 9, **characterized in that** at least one fastening jaw (2) can be mounted on the circumferential surface of the intermediate element (35).

11. Device as in claim 9 or 10, **characterized in that** the intermediate element (35) is split on one side along the centre axis (7) by means of a continuous slot (39).

12. Device as in claim 9 or 10, **characterized in that** the intermediate element (35) is provided with a continuous slot (39) on two mutually opposite sides along the centre axis (7).

13. Device as in one of the claims 9 to 12, **characterized in that** the intermediate element (35) is compressible by the clamping force of the jaw (2) and can thus be locked in position on the central member (8).

14. Device as in one of the claims 1 to 13, **characterized in that** the outer parts (16, 17) are made out of aluminum.

15. Device as in one of the claims 1 to 13, **characterized in that** the outer parts (16, 17) are made out of steel.

16. Device as in one of the claims 1 to 13, **characterized in that** the outer parts (16,17) are made out of a carbon-fiber material.

17. Device as in one of the claims 1 to 13, **characterized in that** the outer parts (16, 17) are made out of titanium.

18. Device as in one of the claims 1 to 17, **characterized in that** the central member (8) is made out of aluminum.

19. Device as in one of the claims 1 to 17, **characterized in that** the central member (8) is made out of steel.

20. Device as in one of the claims 1 to 17, **characterized in that** the central member (8) is made out of a carbon-fiber material.

21. Device as in one of the claims 1 to 17, **characterized in that** the central member (8) is made out of titanium.

22. Device as in one of the claims 1 to 21, **characterized in that** the outer surfaces of the outer parts (16, 17) extending coaxially with the centre axis (7) are rotationally symmetrical.

23. Device as in one of the claims 1 to 22, **characterized in that** the outer cross section of the central member (8) is polygonal and that the outer parts (16, 17) have a correspondingly matching polygonal inner cross section.

## Revendications

1. Dispositif de fixation externe de fragments d'os cassés, lequel comprend un élément de jonction longitudinal (1) avec un axe central (7), dans lequel
A) l'élément de jonction (1) est télescopable dans la direction de cet axe central (7) tout en étant bloqué en rotation ;
B) des mâchoires (2) peuvent être disposées de manière amovible sur l'élément de jonction (1) pour la fixation de vis à os (3) ;
C) l'élément de jonction (1) comprend un segment central (8) et deux segments externes (16 ; 17) pouvant coulisser le long de l'axe central (7) sur le segment central (8) ; et
D) l'élément de jonction (1) peut être rallongé des deux côtés au moyen des segments externes (16 ; 17), lesquels sont disposés de manière à pouvoir être déployés et rétractés au niveau des extrémités opposées (25 ; 26) du segment central (8) ; **caractérisé en ce que**
E) la position axiale des segments externes (16 ; 17) par rapport au segment central (8) peut être ajustée et bloquée par des moyens de transport (19 ; 20) qui sont disposés à l'intérieur des segments externes (16 ; 17).

2. Dispositif selon la revendication 1, **caractérisé en ce que** le segment central (8) d'une part et les segments externes (16 ; 17) d'autre part sont composés de différents matériaux, et chaque segment (8 ; 16 ; 17) est interchangeable et la rigidité de l'élément de jonction (1) dans son ensemble peut donc être ajustée en assemblant des segments faits de différents matériaux (8 ; 16 ; 17).

3. Dispositif selon la revendication 1 ou 2, **caractérisé en ce que** les segments externes (16 ; 17) présentent le même diamètre sur toute leur longueur.

4. Dispositif selon l'une quelconque des revendications 1 à 3, **caractérisé en ce que** les segments externes (16 ; 17), lesquels sont disposés au niveau des extrémités opposées (25 ; 26) du segment central (8), sont déployables et rétractables indépendamment l'un de l'autre.

5. Dispositif selon l'une quelconque des revendications 1 à 4, **caractérisé en ce que** l'enveloppe externe du segment central (8) est réalisée sous forme d'arbre cannelé, et les segments externes (16 ; 17) présentent, en coupe transversale, une forme interne correspondant à celle-ci.

6. Dispositif selon l'une quelconque des revendications 1 à 5, **caractérisé en ce que** les moyens de transport (19 ; 20) sont des broches de transport, lesquelles peuvent être vissées et dévissées dans le segment central (8).

7. Dispositif selon la revendication 6, **caractérisé en ce que** les moyens de transport (19 ; 20) réalisés sous forme de broches de transport peuvent être bloqués par des embouts (30 ; 38) pouvant être vissés dans les segments externes (16 ; 17).

8. Dispositif selon la revendication 7, **caractérisé en ce qu'**au moins un embout (30 ; 38) pouvant être vissé dans les segments externes (16 ; 17) est réalisé sous forme d'embout de dynamisation avec un anneau élastique (40), et **en ce que** la dynamisation consiste à pouvoir relâcher, millimètre par millimètre, le serrage axial d'au moins un moyen de transport (19 ; 20) par un devissage graduel du au moins un embout (30 ; 38), si bien qu'au moins un segment externe (16 ; 17) peut se déplacer axialement sur une distance maîtrisée et que l'on obtient ainsi une dynamisation de la fracture.

9. Dispositif selon l'une quelconque des revendications 1 à 8, **caractérisé en ce qu'**au moins un élément intermédiaire (35) peut être inséré sur le segment central (8) entre les segments externes (16 ; 17), lequel élément peut coulisser et est bloqué en rotation sur le segment central (8) et peut être bloqué dans une position souhaitée sur le segment central (8).

10. Dispositif selon la revendication 9, **caractérisé en ce qu'**au moins une mâchoire (2) peut être mise en place sur la circonférence de l'élément intermédiaire (35).

11. Dispositif selon la revendication 9 ou 10, **caractérisé en ce que** l'élément intermédiaire (35) présente, sur un côté le long de l'axe central (7), une fente continue (39).

12. Dispositif selon la revendication 9 ou 10, **caractérisé en ce que** l'élément intermédiaire (35) présente, sur deux côtés opposés le long de l'axe central (7), une fente continue (39).

13. Dispositif selon l'une quelconque des revendications 9 à 12, **caractérisé en ce que** l'élément intermédiaire (35) peut être compressé par la force de serrage de la mâchoire (2) et être ainsi bloqué sur le segment central (8).

14. Dispositif selon l'une quelconque des revendications 1 à 13, **caractérisé en ce que** les segments externes (16 ; 17) sont faits d'aluminium.

15. Dispositif selon l'une quelconque des revendications 1 à 13, **caractérisé en ce que** les segments externes (16 ; 17) sont faits d'acier.

16. Dispositif selon l'une quelconque des revendications 1 à 13, **caractérisé en ce que** les segments externes (16 ; 17) sont faits d'un matériau à base de fibres de carbone.

17. Dispositif selon l'une quelconque des revendications 1 à 13, **caractérisé en ce que** les segments externes (16 ; 17) sont faits de titane.

18. Dispositif selon l'une quelconque des revendications 1 à 17, **caractérisé en ce que** le segment central (8) est fait d'aluminium.

19. Dispositif selon l'une quelconque des revendications 1 à 17, **caractérisé en ce que** le segment central (8) est fait d'acier.

20. Dispositif selon l'une quelconque des revendications 1 à 17, **caractérisé en ce que** le segment central (8) est fait d'un matériau à base de fibres de carbone.

21. Dispositif selon l'une quelconque des revendications 1 à 17, **caractérisé en ce que** le segment central (8) est fait de titane.

22. Dispositif selon l'une quelconque des revendications 1 à 21, **caractérisé en ce que** les surfaces externes des segments externes (16 ; 17) s'étendant coaxialement à l'axe central (7) sont symétriques en rotation.

23. Dispositif selon l'une quelconque des revendications 1 à 22, **caractérisé en ce que** le segment central (8) présente, en coupe transversale, une forme externe polygonale, et les segments externes (16 ; 17) présentent, en coupe transversale, une forme interne polygonale correspondant à cette forme externe polygonale.
